# EUROPEAN PATENT APPLICATION

(11) **EP 4 767 821 A1**
(43) Date of publication of application: **01.07.2026**
(21) Application number: 25227799.1
(22) Date of filing: 30.12.2025
(51) Int. Cl.: A01M 1/22, A61L 2/104, B62D 49/06

(54) **AGRICULTURE CAPSULE EQUIPMENT WITH UV-C MICROBIOLOGICAL INACTIVATION AND MODULAR GEOMETRIC VARIATION**

(30) Priority: 30.12.2024 PT 2024119967
(71) Applicant: Oprimee - Innovation Design Engineering Solutions, Lda, 3405-155 Oliveira do Hospital (PT)
(72) Inventor: DOS SANTOS ALMEIDA NUNES, JOÃO MIGUEL, 3405-155 OLIVEIRA DO HOSPITAL (PT)
(74) Representative: Patentree

(57) **Abstract**

The present disclosure relates to an agricultural capsule equipment, autonomous or operator-controlled, designed for the microbiological inactivation of pests and pathogens in crops, using controlled UV-C radiation with the modular capacity in terms of height and width and an efficiency protection to ensure that aerosols and insects in aerial form remain inside the equipment and a 360 º inactivation radius. The equipment comprises a low-profile mobile support configured to move along agricultural corridors and an articulated arched structure defining a fully enclosed treatment volume. The autonomous or operator-controlled features an adjustable hydraulic system that allows automatic control of its height and width to adapt to different types of plants, optimizing the application of radiation. The UV-C radiation exposure time and power are adjustable, ranging from 5 to 15 seconds, to ensure effective treatment. At least one UV-C radiation unit is arranged within the enclosed treatment volume, and a control system is configured to regulate displacement speed and UV-C radiation output such that a minimum effective UV-C dose is achieved. An enclosure and flexible sealing elements prevent the emission of UV-C radiation and the dispersion of airborne particles to the exterior. An airflow control system further contributes to containment during operation. The equipment may comprise imaging sensors, including a 360 degree camera system is also used to continuously monitor the plantation, enabling real-time monitoring and automated adjustment of operating parameters. To ensure operator and environmental safety, the radiation unit is protected by a polycarbonate cover that blocks UV-C radiation emissions, and an airflow control system is integrated to prevent the dispersion of contaminating particles during the equipment's movement. The technology aims to reduce the use of chemical pesticides, enabling effective microbiological control, and promoting more sustainable agriculture with a low environmental impact.

## Description

### TECHNICAL FIELD

The present disclosure relates to an agriculture capsule equipment with UV-C microbiological inactivation and modular geometric variation. The technology relates to the field of agricultural technology and specifically to an automated UV-C radiation application equipment for microbiological control of pests and pathogens in crops, including other organisms such us, insects, larvae and others.

### BACKGROUND

Crops as well as their production are crucial to both the sustainability of natural ecosystems and the food security of human populations. As the world's population grows, and with increasing urbanization and climate change posing additional challenges, it is increasingly important to improve plant protection, accelerate plant breeding and ensure that agricultural practices are sustainable and low in carbon dioxide emissions. One promising solution to these challenges is the advancement of plant phenotyping technologies and precision agriculture.

Crop pests and diseases have been significant threats to agricultural production, negatively affecting farmers' incomes, the development of modern agriculture and global production. Ensuring the quality and safety of agricultural products depends heavily on food traceability. Early identification, monitoring and control of diseases and pests are essential to prevent large-scale infestations, maintain crop quality and minimize environmental pollution caused by pesticide residues.

In recent decades, major technological advances in agriculture have drastically transformed various processes in crop and livestock production. These advances mainly focus on minimizing operational and production costs, reducing environmental impact and optimizing the production cycle. However, agricultural operations in the field are complex, requiring a number of issues to be addressed for an effective transition to autonomous or operator-controlled systems.

An autonomous or operator-controlled solution must meet very specific requirements, such as being lightweight, compact, autonomous, intelligent, able to communicate, safe and customizable to perform tasks efficiently. The relatively small size of autonomous machines, compared to conventional tractors and implements, helps to reduce soil-related problems, particularly soil erosion and soil compaction. The advent of autonomous or operator-controlled systems is helping to eradicate hunger and malnutrition in a sustainable way, conserving and restoring ecosystems and natural resources. Consequently, the use of intelligent machines and robots in agriculture is receiving increasing attention.

Autonomous or operator-controlled systems can significantly increase agricultural productivity by optimizing the efficiency of farming practices. Additionally, these autonomous or operator-controlled have the potential to remove humans from dangerous environments and solve labor shortages in seasonal activities. By assessing complex plant characteristics such as growth, development, resistance, physiology and ecology, we can develop high-yielding, stress-tolerant crop varieties that adapt to future climatic conditions and resist pests and diseases.

In recent years, many agricultural autonomous or operator-controlled have been designed and developed to manage crops, plants, livestock and fish. These autonomous or operator-controlled can autonomously perform actions such as harvesting, selective picking and pruning. However, despite these promising advantages, agricultural autonomous or operator-controlled face significant challenges, which include adaptation and proximity to the plant, detection and classification of pests, and the precise application of inputs, along with safety issues. Many of these challenges are associated with the vision system, the autonomous or operator-controlled actuation system, the navigation system in semi-structured agricultural environments, and the intelligence required to control both the autonomous or operator-controlled platform and the implements.

It is therefore imperative that future solutions focus on safety aspects to protect workers, crops and the environment, namely in terms of their potential to minimize the use of chemicals, soil erosion and contribute to the advancement of sustainable agricultural practices. In addition, the economic aspects must be looked at in depth in relation to the practical viability of agricultural autonomous or operator-controlled, as farmers will only invest in them if the investment is profitable over a reasonable period of time.

These facts are disclosed in order to illustrate the technical problem addressed by the present disclosure.

### GENERAL DESCRIPTION

The present disclosure relates to an agriculture capsule equipment with uv-c microbiological inactivation and modular geometric variation.

The present disclosure comprises an agriculture capsule equipment for pest and pathogen control comprising a hydraulic system for adjusting the height and width of the capsule; a set of proximity and navigation sensors configured for autonomous movement and environmental mapping; at least one UV-C radiation unit configured to emit targeted radiation for pest and pathogen inactivation.

The present disclosure relates to intelligent agriculture capsule equipment, designed to carry out the microbiological inactivation of pathogens and deaths of insects, larvae, and others, in plants through controlled UV-C radiation, reducing the use of pesticides and minimizing environmental impact. The equipment is characterized by a highly adaptable and intelligent system, which allows it to carry out precise and efficient operations on different types of crops.

The main innovation of this technology is the application of UV-C radiation, in a modular hydraulic system, in which the power and exposure time are controlled according to the characteristics of the plants and the environmental conditions. This equipment allows for the effective inactivation of pathogenic microorganisms while preserving plant health.

The intelligent agriculture capsule equipment features an adjustable hydraulic system that allows it to automatically modify its height and width, adapting to the structure of the plants in real time, ensuring effective application of radiation. In addition, an adjustable stop ensures that the equipment approaches the shrub structure precisely and without damaging the plants.

The present disclosure relates to an agriculture capsule equipment for pest and pathogen control of crops comprising: a low-profile mobile support for moving along agricultural corridors; an articulated arched structure to be coupled to the low-profile mobile support, configured to form a compartment for receiving the crop; a hydraulic system configured to form a compartment defining an enclosed UV-C treatment volume for receiving the crop, and adjust the articulated arched structure height and/or width according to the crop to be treated; a set of proximity and navigation sensors configured for autonomous movement and environmental mapping; at least one UV-C radiation source arranged in an interior surface of the articulated arched structure, configured to emit targeted radiation to plants for pest and pathogen inactivation; an enclosure surrounding the articulated arched structure lateral and top parts, for preventing the emission of UV-C radiation outside the compartment; a control system configured to control a displacement speed of the mobile support and UV-C emitted by the at least one UV-C radiation unit.

In an embodiment, the control system is configured to determine the displacement speed and/or the operating power of the UV-C radiation unit based on at least one of an effective exposure time determined from a length of the enclosed treatment volume and the displacement speed, and an estimated UV-C irradiance within the enclosed treatment volume, such that the minimum UV-C dose exceeds a predefined threshold value.

In an embodiment, the equipment further comprises an airflow control system configured to generate a pressure differential between the compartment and an exterior environment to inhibit outward dispersion of airborne particles during operation.

In an embodiment, the equipment further comprising at least one imaging sensor configured to monitor the treated corridor and to provide input to the control system.

In an embodiment, the enclosure surrounding the articulated arched structure lateral and top parts is made of polycarbonate, steel, iron, zinc or stainless steel.

To ensure operator's and the environment's safety, the UV-C radiation unit is protected by a structural material with insolation capacity and cover, such us, steel, iron, zinc, stainless steel, polycarbonate, preventing radiation from being emitted outside. The intelligent agriculture capsule equipment also incorporates an airflow control system and a strips of flexible material, such as polycarbonate, acrylic and others, in the tops of the capsule, which directs the airflow in such a way as to prevent the dispersion of pathogenic particles to untreated areas, preventing cross-contamination.

In addition, the intelligent agriculture capsule equipment is equipped with a 360° camera system for continuous monitoring, allowing for automatic adjustments during operation, based on the images captured. This vision system provides real-time monitoring of the effectiveness of the application and allows treatments to be optimized.

The disclosed technology proposes an innovative and sustainable solution for reducing pesticides in agriculture and improve the prevention diseases in plants, using precision technologies and artificial intelligence, which contributes to more sustainable agriculture, with less environmental impact and greater food safety.

The disclosure relates to an intelligent agriculture capsule equipment, designed to treat plants with UV-C radiation in a controlled manner, with the aim of microbiologically inactivating pathogens, deaths of larvae and insects, and reducing the use of chemical pesticides.

In an embodiment, the intelligent agriculture capsule equipment is equipped with an adjustable hydraulic system to automatically control its height and width, adapting to the size and structure of the plants.

In an embodiment, the power of the UV-C radiation and the exposure time of intelligent agriculture capsule equipment are precisely controlled, varying between 5 and 15 seconds, based on the needs of each plant and the environmental conditions.

In an embodiment, the intelligent agriculture capsule equipment contains a 360° camera system that allows continuous monitoring and real-time adjustment of the plantation, guaranteeing the effectiveness of the treatment.

In an embodiment, the intelligent agriculture capsule equipment is protected by a polycarbonate cover, which prevents radiation from being emitted outside the device, guaranteeing the safety of the operators and the environment, characterized with a closed and flexible system made up with strips of flexible material, with a UVC-insulating characteristic, so that they don't pass to the outside and endanger the possible presence of people nearby, such as polycarbonate, acrylic and others, will permit.

In an embodiment, the intelligent agriculture capsule equipment prevents the dispersion of pathogenic particles during its movement, and an air flow control system is integrated to prevent contamination of the treated areas with the strips of flexible material.

It is disclosed an agriculture capsule equipment for pest and pathogen control comprising a hydraulic system for adjusting the height and width of the capsule; a set of proximity and navigation sensors configured for autonomous movement and environmental mapping; at least one UV-C radiation unit configured to emit targeted radiation for pest and pathogen inactivation.

In an embodiment, the hydraulic system of the agriculture capsule equipment comprises hydraulic cylinders controlled by actuators and sensors, enabling dynamic adjustment of the height and width of the capsule in response to plant dimensions and topography.

In an embodiment, the at least one UV-C radiation unit of the agriculture capsule equipment emits radiation in the range of 190 to 250 nm, preferably from 253 to 255 nm, with adjustable power and exposure time between 5 and 15 seconds, determined by a control algorithm.

In an embodiment, the agriculture capsule equipment further comprises an internal reflector configured to maximize the efficiency of the at least one UV-C radiation unit by directing it onto specific areas of the plants.

In an embodiment, the agriculture capsule equipment further comprises an airflow control system that generates a containment zone around the treated area to prevent the dispersion of pathogenic particles or contaminants during operation.

In an embodiment, the airflow control system of the agriculture capsule equipment comprises high-powered fans that dynamically adjust airflow based on the speed of the capsule and local wind intensity.

In an embodiment, the agriculture capsule equipment further comprises at least one 360° camera for real-time crop imaging, enabling the identification of pest-affected areas and automated adjustment of operating parameters.

In an embodiment, the camera system of the agriculture capsule equipment integrates a computer vision system to map the plantation and ensure complete coverage while avoiding untreated areas.

In an embodiment, the UV-C radiation unit of the agriculture capsule equipment is enclosed within a structure, preferably a polycarbonate structure that allows the transmission of radiation required for treatment while blocking external UV-C emission to ensure operator and environmental safety.

In an embodiment, the agriculture capsule equipment further comprises flexible strips of polycarbonate, acrylic, or similar materials to prevent airflow between the interior and exterior of the capsule, maintaining an airtight environment for efficient microorganism inactivation.

In an embodiment, the agriculture capsule equipment further comprises a modular geometric system to facilitate adjustment of dimensions for compatibility with various plantation layouts.

In an embodiment, the control algorithm of the agriculture capsule equipment determines UV-C radiation power and exposure time based on pest density, plant type, and required treatment intensity.

In an embodiment, the agriculture capsule equipment further comprises protective mechanisms, including material insulation and shielding, to prevent accidental exposure to UV-C radiation.

In an embodiment, the hydraulic system of the agriculture capsule equipment adjusts in response to uneven ground topography to ensure stability and consistent proximity to plants.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of an intelligent agriculture capsule equipment.
**Figure 2****:** Schematic representation of the side (i) and front (ii) views of an embodiment of an intelligent agriculture capsule equipment.
**Figure 3****:** Schematic representation of the intelligent agriculture capsule equipment's hydraulic system (1) of an embodiment of an intelligent agriculture capsule equipment.
**Figure 4****:** Schematic representation of a 360° image collection cameras (5) in an embodiment of an intelligent agriculture capsule equipment.

### DETAILED DESCRIPTION

The present disclosure relates to an agriculture capsule equipment with UV-C microbiological inactivation and modular geometric variation.

It is disclosed an agriculture capsule equipment for pest and pathogen control of crops comprising:
a low-profile mobile support for moving along agricultural corridors;
an articulated arched structure to be coupled to the low-profile mobile support, configured to form a compartment for receiving the crop;
a hydraulic system configured to adjust the articulated arched structure height and/or width according to the crop to be treated ;
a set of proximity and navigation sensors configured for autonomous movement and environmental mapping;
at least one UV-C radiation source arranged in an interior surface of the articulated arched structure, configured to emit targeted radiation to plants for pest and pathogen inactivation;
an enclosure surrounding the articulated arched structure lateral and top parts, for preventing the emission of UV-C radiation outside the compartment;
a control system configured to control a displacement speed of the mobile support and UV-C emitted by the at least one UV-C radiation unit.

In an embodiment, the capsule defines a fully enclosed UV-C treatment volume configured such that external UV-C irradiance during operation is substantially zero.

In an embodiment, the control system is configured to adjust at least one of the displacement speed or the UV-C radiation output in response to changes in capsule geometry, such that the minimum UV-C dose is maintained despite variations in plant height or corridor width.

In an embodiment, the enclosure surrounding the articulated arched structure lateral and top parts is made of polycarbonate, steel, iron, zinc or stainless steel.

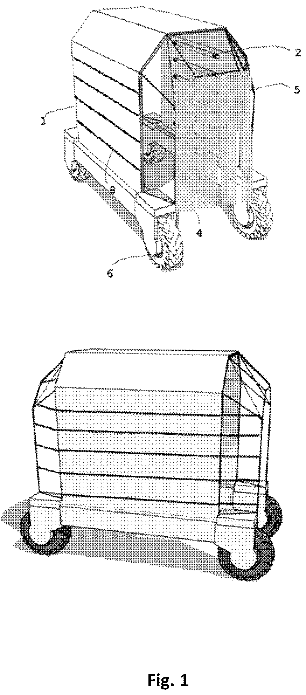

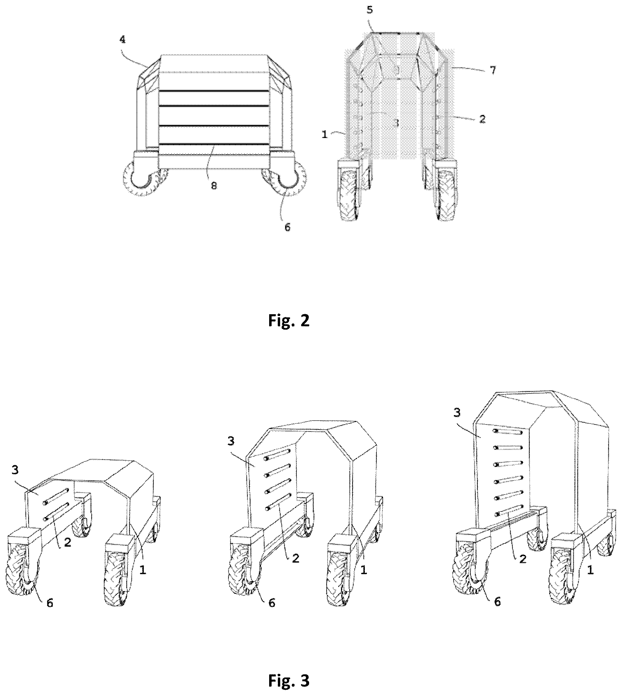

The general concept of the technology is shown in Fig. 1, which comprises an intelligent agriculture capsule equipment, containing an autonomous movement and application unit, made up of a compact and robust structure. The equipment's capsule is constructed from light and resistant materials in order to guarantee durability and low resistance to movement. It comprises a hydraulic system (1), at least one UV-C lamp (2), an internal reflector (3), a structure (4) and 360° image collection camera (5). The intelligent agriculture capsule equipment is moved by wheels powered by an internal motor (6). To decrease the flux of air this equipment has strips of flexible material, such as polycarbonate, acrylic and others, which prevent air from circulating from the inside of the equipment to the outside, in order to make it efficient since many of the microorganisms and insects are in the form of aerosols and air, and thus allow them to remain in the area of action of the equipment and carry out their inactivation and death respectively. This technology comprises a modular geometric system to adapt the height and width of the equipment by a hydraulic system with the capacity to adapt in function of the plant dimensions (8) . The side (i) and front (ii) views of the intelligent agricultural capsule equipment are represented in Fig. 2.

In an embodiment, the intelligent agriculture capsule equipment is designed to operate in plantations of various sizes, such as vegetable gardens, vineyards or fields with bushy plants, and moves autonomously, avoiding damage to the plant. Proximity and navigation sensors allow the intelligent capsule equipment for agriculture to map the environment and move efficiently between rows of plants, carrying out treatments without the need for human intervention.

In an embodiment, the intelligent agriculture capsule equipment is equipped with a hydraulic system (1) and (8), represented in greater detail in Fig. 3, that allows it to dynamically adjust the height and width of the device in response to the topography of the ground and the plant size to be treated. This adjustment is crucial for the equipment to get properly close to the plants, ensuring that the UV-C radiation is applied directly to the leaves, trunks and other plant organs that could be affected by pests and pathogens.

In an embodiment, the hydraulic system (1) and (8) consists of hydraulic cylinders controlled by actuators, which adjust the height of the equipment depending on the density of the plantation. Control is via a system of sensors, which detect the distance between the equipment and the plants, automatically adjusting the parameters to maintain an efficient and safe application.

In an embodiment, the intelligent agriculture capsule equipment is equipped with a UV-C radiation unit with at least one specific UV-C lamp (2) for the 190 to 250 nm and/or 253 to 255 nm range, proven to inactivate fungi, bacteria and viruses present on plants. The radiation unit is designed to emit radiation in a targeted and controlled manner, with adjustable power and an exposure time of between 5 and 15 seconds. Exposure is determined by a control algorithm that takes into account the density of pests, the type of plant and the intensity of radiation required. This part is reinforced by the existence of strips of flexible material on the tops of the equipment (5) that insulate against the passage of UV-C radiation, which on the one hand means that there is no flux of air for outside and an airtight environment inside the equipment and protects the possible presence of people in the proximity.

In an embodiment, the UV-C radiation unit is equipped with an internal reflector (3) that maximizes radiation efficiency, ensuring that the radiation is concentrated on the plant's areas of interest. The radiation unit is installed inside a protective polycarbonate capsule (4), which allows the radiation required for treatment to pass through, but prevents the external emission of UV-C radiation, guaranteeing the safety of operators and the environment.

In an embodiment, during the movement of the intelligent agriculture capsule equipment and the application of UV-C radiation, there is a risk of pathogenic particles or contaminants being dispersed through the air, which could compromise the effectiveness of the treatment. To mitigate this risk, the equipment is equipped with an airflow control system, which creates a containment zone around the treated area.

In an embodiment, the air control system works via high-powered fans located at the rear and side of the intelligent agriculture capsule equipment, which generate a directed air flow that pushes pathological particles or contaminants away from the treated area. This flow control is dynamic, adjusting to the speed of the equipment's movement and the intensity of the local wind, ensuring that the particles do not disperse to adjacent areas.

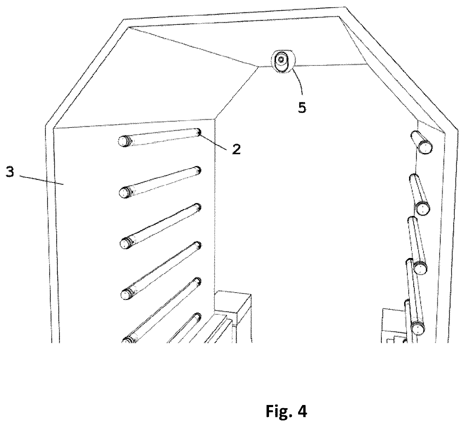

In an embodiment, the intelligent agriculture capsule equipment is equipped with 360° cameras (5), represented in greater detail in Fig. 4, which capture images of the entire crop in real time. These images are processed by a computer vision system that identifies areas affected by pests and microorganisms, allowing the equipment to automatically adjust its operating parameters, such as UV-C power, exposure time, and movement. In addition, the camera system helps to map the plantation and plan the equipment's route, ensuring full coverage and avoiding untreated areas.

In an embodiment, the intelligent agriculture capsule equipment is designed to operate safely for both humans and the environment. The UV-C radiation unit is completely surrounded by a polycarbonate cover, a material that blocks any UV-C radiation emitted outside the device. The cover is specially designed to allow the transmission of the radiation needed for treatment, but block its dispersion into the environment, guaranteeing the safety of operators and preventing exposure to harmful radiation.

Figure 1 illustrates a schematic representation of an embodiment of an intelligent agriculture capsule equipment comprising a hydraulic system (1) that allows height and width adaptation, at least one UV-C lamp (2) for microbiological inactivation, an internal reflector (3), a polycarbonate structure (4) and 360° image collection camera (5). The intelligent agriculture capsule equipment is moved by wheels powered by an internal motor (6).

Figure 2 illustrates the side (i) and front (ii) views of an embodiment of the intelligent agricultural capsule equipment.

Figure 3 illustrates in more detail the intelligent agriculture capsule equipment's hydraulic system (1), which can be adapted to each type of plant, based on its size (height and width).

Figure 4 illustrates the representation of a 360° image collection cameras (5) in an embodiment of an intelligent agriculture capsule equipment for the measurement of crops.

The disclosed technology introduces a set of critical technical parameters that are configured and controlled in a coordinated manner in order to ensure effective microbiological inactivation, operational safety and industrial applicability in agricultural environments. These parameters include the required UV-C dose expressed in mJ/cm², the geometry of the tunnel or capsule, the relationship between displacement speed and exposure time, the characteristics of the airflow including air velocity, volumetric flow rate, differential pressure, precise flow direction and particle dynamics, as well as safety mechanisms for human protection including fully hermetic enclosure, maximum external irradiance limits, presence detection sensors, maximum operating time, and a control algorithm defining inputs, outputs, operating limits, control logic and parameter thresholds.

The disclosed technology relates to an agricultural capsule equipment integrating an enclosed UV-C tunnel or capsule, aerodynamic flow control, sensors and a control algorithm, intended for microbiological inactivation in agricultural environments. The equipment is suitable for applications including treatment of air, surfaces, tools, packaging or passage corridors, and operates without human exposure to UV-C radiation, thereby providing an alternative or complementary solution to chemical inputs. The equipment's architecture, tunnel or capsule configuration, airflow path and control modules are illustrated in the accompanying figures.

The microbiological inactivation efficacy of the equipment is determined by the accumulated UV-C dose, which is a function of the effective irradiance and the exposure time. The equipment is configured to operate with engineering dose ranges ensuring generic microbiological reduction without reference to specific organisms. A minimum effective dose in the range of 10 to 30 mJ/cm² is considered functional, while preferred operation provides a margin corresponding to doses equal to or greater than 30 mJ/cm². The equipment is configured to guarantee a minimum effective dose above a predefined threshold value through coordinated control of irradiance, exposure time and geometry.

The geometry of the tunnel or capsule is configured to increase residence time and dose uniformity without increasing the overall volume of the equipment. The effective treatment path length is typically from 1.5 m to 6.0 m, preferably from 2.0 m to 4.0 m. The cross-section may be circular or rectangular and incorporates smooth curvature with a minimum radius equal to at least 1.5 times the hydraulic diameter, thereby avoiding sharp angles exceeding 90 degrees. The internal architecture preferably follows a sinuous path, and internal surfaces are coated or manufactured with UV-C reflective materials in order to enhance irradiance efficiency.

For a given UV-C irradiance, the effective exposure time is determined by the displacement speed of the agricultural capsule equipment or, in air-treatment configurations, by the effective air velocity within the UV-C zone. Typical operating velocities in the UV-C zone range from 0.2 m/s to 1.0 m/s. The equipment is configured to automatically adjust the displacement speed when irradiance decreases, for example due to lamp ageing or increased dose requirements, in order to maintain the minimum effective dose.

The airflow is dimensioned according to the capacity of the agricultural capsule equipment and the scale of the application. Differential pressure is optimized to minimize losses in curves and diffusers. The airflow direction is configured to be substantially parallel to the UV-C sources in order to avoid frontal incidence and shadowing effects. Particle dynamics are controlled such that mixing occurs upstream and downstream of the UV-C exposure zone, while the exposure zone itself is maintained under predominantly laminar flow conditions. Preferably, the Reynolds number within the UV-C zone is maintained below 2300, with controlled transition where required.

The UV-C sources are arranged substantially parallel to the airflow and distributed symmetrically along the effective treatment path. The distance between the airflow and the UV-C sources is typically from 50 mm to 150 mm. The equipment is configured to maintain irradiance uniformity within ±20% across the treated cross-section. Internal surfaces exhibit a UV-C reflectance of at least 0.70, preferably equal to or greater than 0.85, thereby increasing effective irradiance and compensating for ageing and contamination. Suitable materials include polished or anodized aluminum, mirrored stainless steel and technical PTFE.

The equipment operates with a fully enclosed UV-C treatment module that is completely hermetic to UV-C radiation. External irradiance during operation is approximately zero, with no detectable leakage. The enclosure incorporates sensors for door position, presence detection and structural integrity. Interlocks are configured to immediately deactivate UV-C sources in the event of enclosure opening, presence detection or system failure. Maximum operating time is limited by the control algorithm and sensor states, such that the equipment includes enclosure and interlock means configured to prevent any emission of UV-C radiation to the exterior.

In an embodiment, the equipment comprises a control algorithm implemented in the agricultural capsule equipment. Inputs include airflow rate or velocity, pressure, UV-C irradiance or ageing proxies, internal temperature, door or presence status and cumulative UV operating hours. Outputs include UV-C power control, fan speed adjustment, activation of mixing valves or diffusers where applicable, and alarm or safety states. The control logic verifies safety conditions prior to operation, calculates a target UV-C dose, adjusts speed and UV-C power to guarantee the minimum dose, maintains appropriate flow regimes within the UV-C zone, compensates for UV source ageing, and terminates operation upon reaching predefined limits or detecting faults.

In an embodiment, the equipment is applicable to agricultural passage corridors, greenhouse air treatment, physical disinfection of surfaces, packaging or tools, and mobile treatment modules. The technical effect achieved is a reduction of microbiological pressure by physical means, resulting in reduced reliance on chemical pesticides or herbicides, improved productivity and enhanced sustainability. The equipment is scalable by adjusting airflow and operational parameters according to the specific agricultural scenario.

Engineering validation includes verification of UV-C dose and exposure time through radiometric measurement, airflow mapping through velocity and pressure measurements, irradiance uniformity verification across the treated section, safety testing confirming absence of external UV-C irradiance, and robustness testing including thermal cycling, vibration and extended UV operating hours.

In an embodiment, the equipment is applied in agricultural corridors in order to reduce the use of chemical pesticides or herbicides while maintaining crop productivity and quality. The effect of pesticide reduction is evaluated using measurable agricultural metrics including the treatment frequency index or number of treatments per season, the amount of active substance applied per hectare, the cost per hectare associated with phytosanitary products and application operations, and the resulting productivity expressed in kilograms per hectare together with qualitative crop parameters. In this embodiment, the equipment operates as a physical microbiological inactivation tool that enables a reduction in the number of chemical applications in treated corridors, while maintaining productivity within an acceptable variation range.

In a representative proof-of-concept scenario, a baseline agricultural practice is defined in which a crop corridor is subjected to ten chemical treatments per season, corresponding to a total of approximately 1.2 kilograms of active substance per hectare per season. Each treatment involves a combined cost per hectare including the chemical product cost, estimated at approximately thirty euros, labor time corresponding to approximately four hours, estimated at forty euros, and machinery usage and fuel costs estimated at approximately two hundred and fifty euros, resulting in a total cost of approximately three hundred and twenty euros per application per hectare, corresponding to approximately three thousand two hundred euros per hectare per season.

In the same corridor treated using the UV-C microbiological inactivation equipment according to the invention, the number of chemical applications is reduced by approximately thirty percent, corresponding to seven applications per season. The amount of active substance applied per hectare is reduced proportionally to approximately 0.84 kilograms per hectare per season. Under these conditions, the direct economic saving associated with the reduction of chemical treatments is approximately nine hundred and sixty euros per hectare per season, corresponding to approximately thirty percent of the baseline phytosanitary cost, while maintaining crop productivity within a variation of plus or minus five percent relative to the baseline. This embodiment demonstrates that the equipment enables a measurable reduction in chemical inputs through physical microbiological control while preserving agricultural performance parameters.

In an embodiment, the equipment is configured to achieve effective microbiological inactivation by physical means through controlled application of UV-C radiation, with performance evaluated using engineering metrics based on logarithmic reduction of a generic microbiological load. The reduction is expressed as a logarithmic reduction value defined as the base-10 logarithm of the ratio between an upstream microbiological count and a downstream microbiological count, expressed for example in colony forming units per cubic meter or an equivalent measure. This metric provides a quantitative indication of the reduction achieved by the equipment without reference to specific microorganisms.

In this embodiment, the effective UV-C dose applied to the treated volume is calculated as the product of the average effective irradiance within the UV-C exposure zone and the effective exposure time. The exposure time is determined by the effective length of the UV-C treatment zone and the displacement speed of the agricultural automaton or, in air-treatment configurations, by the effective air velocity within the UV-C zone.

In a representative proof-of-concept configuration, a target UV-C dose of approximately 30 mJ/cm² is defined by the control algorithm. For a given effective tunnel length and a defined displacement speed or effective air velocity, the exposure time is calculated as the ratio between the effective length of the treatment zone and the velocity. The average irradiance required to achieve the target dose is then determined by dividing the target dose by the calculated exposure time. The control algorithm is configured to adjust at least one of the irradiance or the velocity in order to ensure that the target dose is achieved at the most unfavourable point within the treated volume.

Under representative field conditions, the equipment is configured to achieve a logarithmic reduction value of at least one to two log units, while under controlled conditions such as laboratory, bench or greenhouse environments, the equipment is configured to achieve a logarithmic reduction value of at least two to three log units. The effective reduction achieved depends on factors including the biological target, the presence of dust, humidity, shading effects and the minimum guaranteed UV-C dose maintained throughout the treated volume, including the peripheral regions corresponding to a 360-degree treatment configuration.

In an embodiment related to agricultural corridors in tree plantations, an agricultural capsule equipment moves along a corridor between rows of trees and incorporates an enclosed treatment module of the tunnel or capsule type. In an embodiment, the tunnel or capsule comprises a physical length of approximately 3 m and is dimensioned to treat corridors having a width from 1.5 m to 3 m and a canopy height from 1 m to 4 m. The equipment comprises UV-C radiation sources arranged so as to provide a 360-degree treatment of the treated volume, and an enclosure configured to prevent any emission of UV-C radiation to the exterior during operation.

In addition to the physical length of the tunnel, the equipment is configured to maintain an effective radiation reach of approximately 1 m in front of and 1 m behind the tunnel, corresponding to a 360-degree treatment effect. The effective exposure length is therefore defined as the sum of the physical tunnel length and twice the effective radiation reach. For a physical tunnel length of approximately 3 m and an effective reach of approximately 1 m on each side, the effective exposure length is approximately 5 m.

In an embodiment, the agriculture capsule equipment operates at a displacement speed corresponding to more than 1 m travelled in 10 seconds. The effective exposure time of the treated volume is determined as the ratio between the effective exposure length and the displacement speed of the equipment or, equivalently, the effective air velocity within the UV-C exposure zone. For typical operating speeds, the resulting exposure times fall within a range suitable to achieve the target UV-C dose defined by the control algorithm.

The effective UV-C dose applied to the treated volume is defined as the product of the average effective irradiance in the exposure zone and the effective exposure time. Considering an engineering target dose defined by the control system, the minimum average irradiance required is calculated by dividing the target dose by the effective exposure time. For the exposure times obtained under the operating conditions described above, the control system determines the corresponding minimum irradiance values and adjusts the UV-C power and/or the displacement speed accordingly in order to ensure that the target dose is achieved.

For proof-of-concept dimensioning, the internal treated surface area of the tunnel is approximated by the perimeter of the transverse cross-section multiplied by the effective tunnel length. For corridors of smaller dimensions, corresponding to a width of approximately 1.5 m and a height of approximately 1 m, a first envelope of treated area is obtained, while for larger corridors corresponding to a width of approximately 3 m and a height of approximately 4 m, a second, larger envelope of treated area is obtained. The required radiant UV-C power is estimated as a function of the target irradiance, the treated surface area and a global optical coupling efficiency factor accounting for geometric losses, shading, ageing of the UV-C sources and surface contamination. This efficiency factor is assumed to lie within a range from 0.15 to 0.35, with a value of 0.25 considered robust for proof-of-concept calculations under agricultural conditions. Based on these assumptions, a corresponding range of radiant UV-C power is defined in order to cover the full operating envelope in terms of corridor width, height and operating speed.

The electrical power associated with the UV-C sources is estimated by dividing the required radiant UV-C power by an electrical-to-useful-UV conversion efficiency, which depends on the emission technology and typically lies in the range from 0.10 to 0.35. Taking into account dust, ageing and other agricultural operating conditions, a preferred electrical power range is selected to provide sufficient margin under extreme scenarios.

The 360-degree treatment criterion is verified by defining a minimum guaranteed UV-C dose at the most unfavourable point of the treated perimeter, at a distance up to the defined effective radiation reach. The equipment integrates sensors and/or calibration procedures configured to map irradiance and dose at multiple angular positions around the treated volume, including positions at 0°, 90°, 180° and 270° as well as along the central axis, and at multiple longitudinal positions corresponding to the entrance, middle and exit of the tunnel. If the measured dose at the most unfavourable point is below the target value, the control system automatically adjusts the displacement speed and/or the UV-C power in order to restore the minimum guaranteed dose.

During agricultural operation, the UV-C module operates within a fully enclosed housing and comprises interlocks configured to immediately deactivate the UV-C sources in the event of opening of the enclosure, detection of human presence or loss of system integrity. This configuration ensures that no UV-C radiation is emitted to the exterior during operation, thereby enabling safe use of the equipment in agricultural environments.

The described examples of embodiments constitute non-limiting embodiments of the claimed equipment and demonstrate, through technical and measurable means, the functional effects associated with the features defined in the claims. The examples demonstrate establishment of a guaranteed minimum effective UV-C dose through cooperation between irradiance, exposure time and geometry, measurable reduction of microbiological load by physical treatment, containment preventing particle escape, and operational safety through enclosure and interlocks. Further examples demonstrate that path geometry and effective exposure length ensure achievement of the target dose even at elevated operating speeds, that automatic adjustment by the control algorithm guarantees the minimum dose at the most unfavourable point of the treated volume, that UV-C source arrangement and reflective surfaces ensure uniform 360-degree treatment without shadow zones, that ageing and contamination are compensated through control adjustments, and that long-term operation under agricultural conditions maintains functional stability.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following dependent claims further set out particular embodiments of the disclosure.

## Claims

1. An agriculture capsule equipment for pest and pathogen control of crops comprising:
a low-profile mobile support for moving along agricultural corridors;
an articulated arched structure to be coupled to the low-profile mobile support, configured to form a compartment for receiving the crop;
a hydraulic system configured to form a compartment defining an enclosed UV-C treatment volume for receiving the crop, and adjust the articulated arched structure height and/or width according to the crop to be treated;
a set of proximity and navigation sensors configured for autonomous movement and environmental mapping;
at least one UV-C radiation unit arranged in an interior surface of the articulated arched structure, configured to emit targeted radiation to the crop for pest and pathogen inactivation;
an enclosure surrounding the articulated arched structure lateral and top parts, for preventing the emission of UV-C radiation outside the compartment;
a control system configured to control a displacement speed of the mobile support and UV-C emitted by the at least one UV-C radiation unit.

2. The agriculture capsule equipment according to the previous claim, wherein the control system is configured to determine the displacement speed and/or the operating power of the UV-C radiation unit based on at least one of an effective exposure time determined from a length of an enclosed treatment volume of the compartment and the displacement speed, and
an estimated UV-C irradiance within the enclosed treatment volume, such that the minimum UV-C dose exceeds a predefined threshold value.

3. The agriculture capsule equipment according to any of the previous claims, further comprising an airflow control system configured to generate a pressure differential sufficient to inhibit outward dispersion between the compartment and an exterior environment to inhibit outward dispersion of airborne particles during operation.

4. The agriculture capsule equipment according to any of the previous claims, further comprising at least one imaging sensor configured to monitor the treated corridor and to provide input to the control system.

5. The agriculture capsule equipment according to any of the previous claims, wherein the hydraulic system comprises hydraulic cylinders controlled by actuators and sensors.

6. The agriculture capsule equipment according to any of the previous claims, wherein the at least one UV-C radiation unit emits radiation in the range of 190 to 250 nm, preferably from 253 to 255 nm, with adjustable power and exposure time between 5 and 15 seconds.

7. The agriculture capsule equipment according to any of the previous claims, wherein the compartment defines a fully enclosed UV-C treatment volume such that external UV-C irradiance during operation is substantially zero and comprises an internal reflector configured to maximize the efficiency of the at least one UV-C radiation unit.

8. The agriculture capsule equipment according to any of the previous claims 3-7, wherein the airflow control system comprises high-powered fans that dynamically adjust airflow based on the speed of the capsule and local wind intensity, preferably the high-powered fans are placed at a rear part or side part of the articulated arch structure.

9. The agriculture capsule equipment according to any of the previous claims, further comprising at least one 360° camera for real-time crop imaging, enabling the identification of pest-affected areas and automated adjustment of operating parameters, preferably the camera is placed at the top of the articulated arched structure, more preferably wherein the camera integrates a computer vision system to map the plantation and ensure complete coverage while avoiding untreated areas.

10. The agriculture capsule equipment according to any of the previous claims, wherein the enclosure surrounding the articulated arched structure lateral and top parts is made of polycarbonate, steel, iron, zinc or stainless steel, or equivalent UV-C shielding materials.

11. The agriculture capsule equipment according to any of the previous claims, further comprising flexible strips of polycarbonate, acrylic, or similar materials to prevent airflow between the interior and exterior of the articulated arched structure, maintaining an airtight environment for efficient microorganism inactivation.

12. The agriculture capsule equipment according to any of the previous claims, further comprising a modular geometric system to facilitate adjustment of dimensions for compatibility with various plantation layouts.

13. The agriculture capsule equipment according to any of the previous claims, wherein the control system determines UV-C radiation power and exposure time based on pest density, plant type, and required treatment intensity, as detected by the imaging sensors.

14. The agriculture capsule equipment according to any of the previous claims, further comprising protective mechanisms, including material insulation and shielding, to prevent accidental exposure to UV-C radiation.

15. The agriculture capsule equipment according to any of the previous claims, wherein the hydraulic system adjusts in response to uneven ground topography to ensure stability and consistent proximity to plants.
